# EUROPEAN PATENT APPLICATION

(11) **EP 4 834 270 A1**
(43) Date of publication of application: **04.11.2026**
(21) Application number: 24910617.0
(22) Date of filing: 05.12.2024
(51) Int. Cl.: A61B 34/35

(54) **SURGICAL ROBOT AND MEDICAL SYSTEM**

(30) Priority: 29.12.2023 CN 202311872535; 27.06.2024 CN 202410854619; 27.06.2024 CN 202410854907
(71) Applicant: Cornerstone Technology (Shenzhen) Limited, Shenzhen Guandong (CN)
(72) Inventor: CAI, Changrong, Shenzhen, Guangdong 518066 (CN); LU, Tao, Shenzhen, Guangdong 518066 (CN)
(74) Representative: Balder IP Law, S.L.
(86) International application number: PCT/CN2024/137184
(87) International publication number: WO 2025/139685

(57) **Abstract**

Provided are a surgical robot and a medical system. The surgical robot includes: an orientation platform, at least two load arms, and at least two setup arms. The at least two load arms coupled to the orientation platform and rotatable about a first axis relative to the orientation platform, each load arm includes a straight section extending in a horizontal direction, and at least one load arm further includes a bent section coupling its straight section with the orientation platform, such that when the at least two load arms are in a gathered state, straight sections of the at least two load arms are aligned side by side and parallel to each other. Each setup arm is coupled to a straight section of an associated load arm and movable relative to the associated load arm along an extension direction of the straight section of the associated load arm.

## Description

### CROSS REFERENCE TO RELATED APPLICATIONS

This application claims priority to Chinese Patent Application No. 202311872535.3, filed on December 29, 2023, Chinese Patent Application No. 202410854907.8, filed on June 27, 2024, and Chinese Patent Application No. 202410854619.2, filed on June 27, 2024, each of which is incorporated by reference herein in its entirety.

### TECHNICAL FIELD

The present disclosure belongs to the technical field of medical devices, and particularly relates to a surgical robot and a medical system.

### BACKGROUND

Surgical robots have advantages such as accurate positioning, stable operation, high dexterity, large working range, and immunity to radiation and infection, and are widely used in various surgeries. A surgical robot controls surgical micro-instruments to perform surgeries, which helps improve the precision of surgeons, solves problems such as hand tremors, fatigue, and neuromuscular feedback of surgeons, and enables surgeons to perform surgical operations in the most comfortable state. The surgical robot has significant value in improving the success rate of surgeries and alleviating patient suffering. In recent years, research on surgical robots has become a new field in medical device applications.

However, existing surgical robot products still have defects such as redundant structural degrees of freedom and high structural complexity. These defects cause difficulties in preoperative setup and inconvenience in performing surgeries.

### SUMMARY

The present disclosure provides a surgical robot and a medical system, so as to solve or improve at least one of the defects of the existing surgical robots, such as redundant degrees of freedom and high structural complexity.

In a first aspect of the present disclosure, a surgical robot is provided. The surgical robot includes an orientation platform, at least two load arms, and at least two setup arms. The at least two load arms are coupled to the orientation platform and rotatable about a first axis relative to the orientation platform. Each respective load arm of the at least two load arms includes a straight section extending in a horizontal direction, and at least one load arm of the at least two load arms further includes a bent section coupling its straight section with the orientation platform, such that when the at least two load arms are in a gathered state, straight sections of the at least two load arms are aligned side by side and parallel to each other. The at least two setup arms are each coupled to a straight section of an associated load arm and movable relative to the associated load arm along an extension direction of the straight section of the associated load arm, where the at least two setup arms have a same minimum radius and a same maximum radius of rotation about a central axis of the orientation platform.

In the above surgical robot, by providing the orientation platform, the load arms and setup arms coupled to the load arms can all perform rotational movements about the orientation platform as a center. Compared with existing multi-arm separate robot configurations, the space occupation of the load arms and the setup arms is saved. Meanwhile, multiple load arms can be arranged on the same orientation platform, thereby reducing the structural complexity.

In a second aspect of the present disclosure, a medical system is provided, including a surgeon console and the above surgical robot.

### BRIEF DESCRIPTION OF THE DRAWINGS

In order to more clearly illustrate the technical solutions of the embodiments of the present disclosure, the drawings required for the embodiments of the present disclosure will be briefly introduced below. For those of ordinary skill in the art, other drawings can also be obtained based on these drawings without creative efforts.
FIG. 1 is a schematic diagram of a medical system according to the present disclosure.
FIG. 2 is a structure view of a surgical robot according to the present disclosure.
FIG. 3 is a principle schematic diagram of a surgical robot.
FIG. 4 is a structure view of an implementation of a connection structure between an orientation platform and load arms of a surgical robot according to the present disclosure.
FIG. 5 is another state diagram of the implementation of FIG. 4.
FIG. 6 is a structure view of an implementation of the surgical robot according to the present disclosure.
FIG. 7 is a structure view of another implementation of the surgical robot according to the present disclosure.
FIG. 8 is a structure view of yet another implementation of the surgical robot according to the present disclosure.
FIG. 9 is a structure view of an implementation of a connection structure between the orientation platform and the load arms of the surgical robot according to the present disclosure.
FIG. 10 is a structure view of another implementation of the connection structure between the orientation platform and the load arms of the surgical robot according to the present disclosure.
FIG. 11 is a structure view of yet another implementation of the connection structure between the orientation platform and the load arms of the surgical robot according to the present disclosure.
FIG. 12 is a structure view of still yet another implementation of the connection structure between the orientation platform and the load arms of the surgical robot according to the present disclosure.
FIG. 13 is a structure view of an implementation of the surgical robot according to the present disclosure.
FIG. 14 is a top view of an orientation platform and a load arm according to an embodiment of the present disclosure.
FIG. 15 is a perspective view of an orientation platform and load arms of the surgical robot according to the present disclosure.
FIG. 16 is a structure view of an implementation of a mechanical motion structure between an orientation platform and a load arm of the surgical robot according to the present disclosure.
FIG. 17 is an enlarged view of part A in FIG. 15.
FIG. 18 is a perspective partial sectional view of the mechanical motion structure between the orientation platform and the load arms shown in FIG. 15.
FIG. 19 is a top sectional view of the mechanical motion structure between the orientation platform and the load arms shown in FIG. 15.
FIG. 20 is a structure view of another implementation of the mechanical motion structure between the orientation platform and the load arms according to the present disclosure.
FIG. 21 is a top sectional view of the mechanical motion structure between the orientation platform and the load arms shown in FIG. 20.
FIG. 22 is a structure view of another orientation platform and load arm of the surgical robot according to the present disclosure.
FIG. 23 is a top view of the mechanical motion structure between the orientation platform and the load arms shown in FIG. 22.
FIG. 24 is a perspective view of the mechanical motion structure between the orientation platform and the load arms shown in FIG. 22.
FIG. 25 is a perspective sectional view of the mechanical motion structure between the orientation platform and a transmission device shown in FIG. 22.
FIG. 26 is a top sectional view of the mechanical motion structure between the orientation platform and the load arms shown in FIG. 22.
FIG. 27 is an exploded perspective view of the transmission device shown in FIG. 26.

### DETAILED DESCRIPTION OF THE EMBODIMENTS

In order to make the objectives, technical solutions, and advantages of the embodiments of the present disclosure clearer, the embodiments of the present disclosure will be described in detail below with reference to the accompanying drawings. However, those of ordinary skill in the art can understand that, in the embodiments of the present disclosure, many technical details are proposed to enable a better understanding of the present disclosure. However, even without these technical details and various changes and modifications based on the following embodiments, the technical solution claimed in the present disclosure can be implemented.

In the embodiments of the present disclosure, terms such as "upper," "lower," "left," "right," "front," "rear," "top," "bottom," "inner," "outer," "middle," "vertical," "horizontal," "transverse" and "longitudinal" indicating orientations or positional relationships are based on the orientations or positional relationships shown in the drawings. These terms are mainly used to better describe the present disclosure and its embodiments, and are not intended to limit that the indicated devices, elements or components must have specific orientations or be constructed and operated in specific orientations.

Moreover, some of the above terms may also indicate other meanings in addition to indicating the orientations or positional relationships. For example, the term "upper" may also indicate a certain attachment relationship or connection relationship in some cases. For those of ordinary skill in the art, the specific meanings of these terms in the present disclosure can be understood according to specific circumstances.

In addition, terms such as "install," "set," "provide," "open," "connect" and "couple" should be understood in a broad sense. For example, the connections may be fixed connections, detachable connections, or integral constructions, may be mechanical connections or electrical connections, may be direct connections or indirect connections through intermediate media, or may be internal communications between two devices, elements or components. For those of ordinary skill in the art, the specific meanings of the above terms in the present disclosure can be understood according to specific circumstances.

It should be noted that, in this text, relational terms such as first and second are only used to distinguish one entity or operation from another entity or operation, and do not necessarily require or imply any such actual relationship or order between these entities or operations. Moreover, the terms "include," "comprise" or any other variants are intended to cover a non-exclusive inclusion, such that a process, method, article or device including a series of elements not only includes those elements, but also includes other elements not explicitly listed, or also includes elements inherent to such process, method, article or device. Without more limitations, an element defined by the phrase "including a..." does not exclude the presence of an additional identical element in the process, method, article or device that includes the element.

The terms "distal" and "proximal" used in the present disclosure are directional terms, which are conventional terms in the field of interventional medical devices, where "distal" indicates an end away from the operator during surgery, and "proximal" indicates an end close to the operator during surgery. In a remotely controlled surgical robot system, "operator" refers to a patient-side robot that holds and actuates a surgical instrument.

Terms "center," "parallel," "vertical" and similar expressions used in the present disclosure do not have to be precise, but may include typical engineering tolerances.

Now, exemplary embodiments according to the present disclosure are described in more detail with reference to the accompanying drawings.

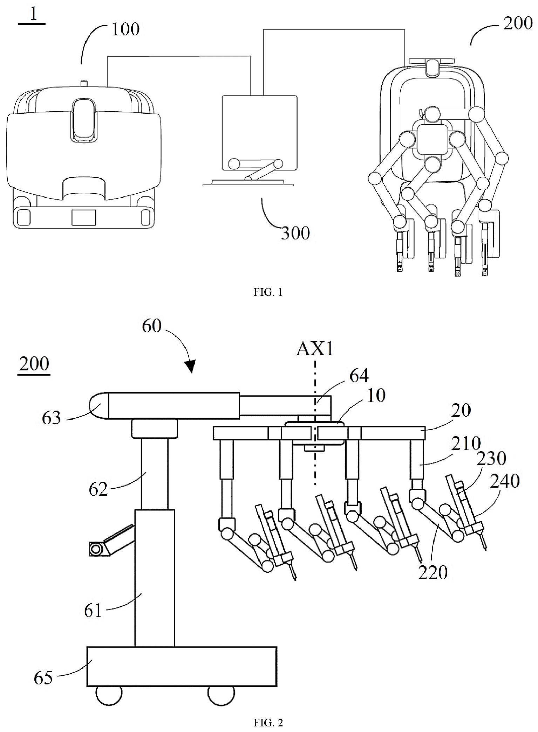

Referring to FIG. 1, an embodiment of the present disclosure provides a medical system 1. The medical system 1 of the present disclosure is a surgical robot system capable of remotely controlling surgery. The medical system may include a surgeon console 100, a surgical robot 200, and an imaging device 300.

The surgeon console 100 has a display unit for presenting the surgical instrument environment, a surgeon's operation control mechanism, an armrest, and the like. The display unit is provided with an observation window for the surgeon to observe, the operation control mechanism is configured such that movements of the operation control mechanism correspond to movements of surgical instruments, and the armrest is configured for placing the surgeon's arm. In addition, the surgeon console 100 further has an additional control switch that is convenient for a hand or foot to touch or press and configured to perform various functional operations to achieve human-computer interaction.

The imaging device 300 has a display screen, an endoscope controller, system electronic equipment, an image processor, and the like.

Referring to FIG. 2, the surgical robot 200 may include multiple mechanical arms, and each mechanical arm may include a setup arm 210 and a manipulator arm 220. The setup arm 210 and the manipulator arm 220 enable a distal end of the mechanical arm to achieve multi-degree-of-freedom (e.g., seven degrees of freedom, which may vary according to different surgical instruments) movement. An instrument arm 230 is disposed at the distal end of the mechanical arm, and an end effector 240 is detachably mounted on the instrument arm 230. The end effector 240 may be an instrument for performing surgical operations, such as an electrocautery, a clamp, or a vascular sealer, or may be a camera for capturing images of the surgical area, such as an endoscope, or may be another surgical instrument.

In some application scenarios, the mechanical arms may be configured to mechanically move around a remote center of motion (RCM). For example, in laparoscopic surgery, the RCM is defined as a port for entering the patient's abdominal cavity during surgery. During the surgery, the mechanical arm is manipulated so that the instrument arm 230 drives the end effector 240 to perform movements such as pitch, yaw, insertion, and rotation. During the movements, a longitudinal axis of the end effector 240 always passes through the RCM, so as to avoid non-surgical damage to the patient's abdominal incision by the end effector 240.

The end effector 240 may include tools for performing surgical operations (e.g., cutting tissue), such as a hook, a spatula, a clamp, and a scissor, or may be an endoscope lens for capturing images.

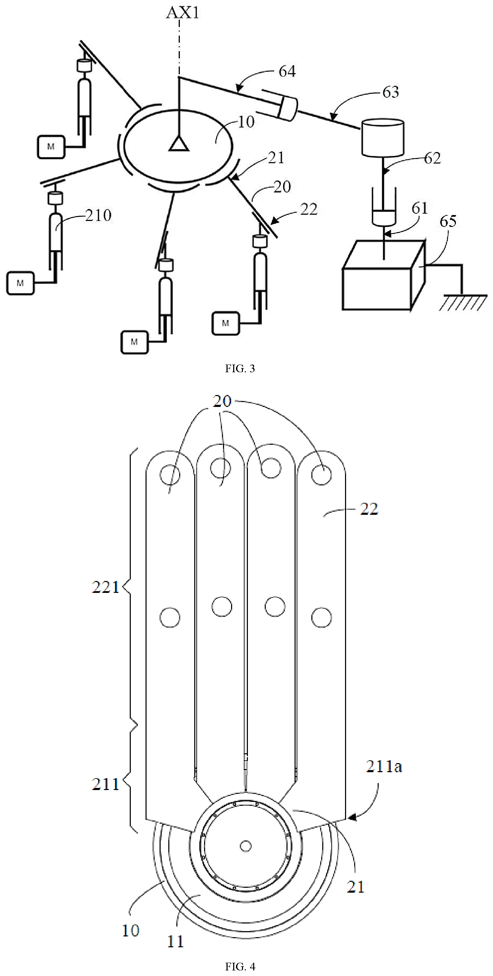

As shown in FIG. 3, the surgical robot 200 of the present disclosure further includes: an orientation platform 10 and at least two load arms 20 coupled to the orientation platform 10. The load arm 20 is coupled to the setup arms 210, and may be considered as part of the mechanical arm.

The orientation platform 10 is a support component for the load arms 20, the setup arms 210, the manipulator arms 220, etc. An entire weight of the load arms 20, the setup arms 210, and the manipulator arms 220 is borne by the orientation platform 10. Substantially, an entire weight of the mechanical arms is borne by the orientation platform 10.

The orientation platform 10 may be configured as an annular component or a component having an annular structure. Each load arm 20 is individually coupled to the orientation platform, and is rotatable relative to the orientation platform 10, so as to adjust positions of the setup arms 210 and the manipulator arm 220 by rotating about the orientation platform 10. A rotation center of each load arm 20 rotating relative to the orientation platform 10 is located on the same rotation axis AX1, which, for example, may be a central axis of the annular component.

In the surgical robot 200 of this embodiment, by providing the orientation platform 10 to support the rotation of the load arms 20, the degrees of freedom of the mechanical arms are simplified, structural redundancy is avoided, overall convenience of using the surgical robot 200 is improved, a control program of the mechanical arms is easy to set, control logic is clear, and accuracy of performing surgery is improved.

In an embodiment, as shown in FIG. 3, each load arm 20 includes a connection end 21 and a load end 22.

The connection end 21 is an end of the load arm 20 coupled to the orientation platform 10, the load end 22 is a free end of the load arm 20, and the load end 22 is configured to connect the setup arm 210. In some embodiments, multiple load arms 20 are arranged radially on the orientation platform 10, and a connection line between the connection end 21 and the load end 22 of each load arm 20 can intersect the rotation axis AX1 of the load arm 20. The load arm 20 as a whole may be substantially linear, or may have a special shape different from the linear shape, but at least partially has an extension length.

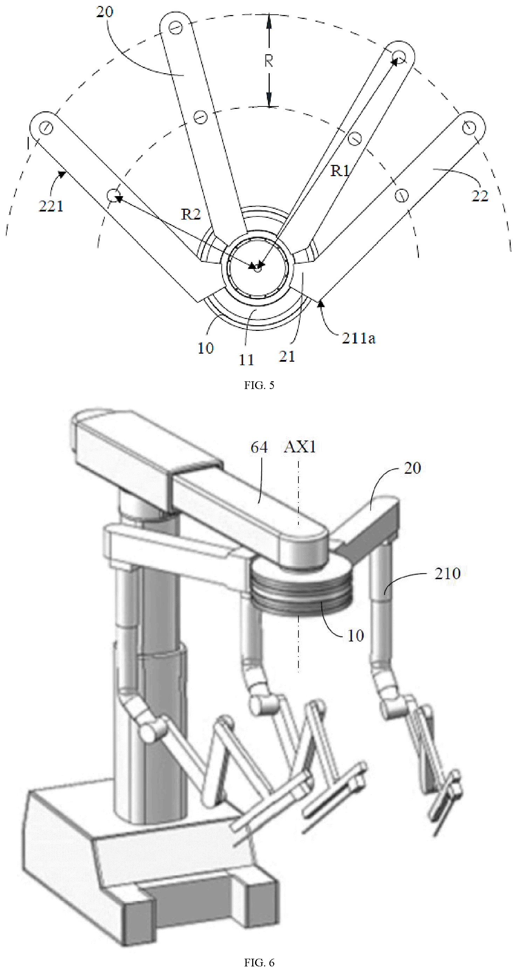

In some situations, for example, the load arms 20 of the surgical robot need to be stored and arranged neatly when in a non-working state, the radially arranged load arms 20 cannot be aligned side by side, and occupy a large space. In some embodiments, referring to FIG. 4, each load arm 20 includes a straight section 221, and the straight sections 221 of the load arms 20 may be aligned side by side. That is, when all load arms are in a gathered state, the straight sections 221 of the load arms 20 are aligned in parallel or side by side. Specifically, referring to FIG. 5, each load arm 20 at least includes a straight section 221 extending to the load end 22. Meanwhile, some load arms 20 may each include a bent section 211 extending to the connection end 21 and a straight section 221 extending to the load end 22. The bent section 211 of each of these load arms 20 includes a bent structure 211a, and the bent structure 211a of each load arm 20 may be different. For example, referring to FIG. 4, taking an even number of load arms 20 as an example, a midline between two load arms 20 is taken as a symmetry axis, where a load arm 20adjacent to the midline has a bent structure 211a with a small bending angle, or may even have no bent structure or bent section, while a load arm 20 farther away from the midline has a bent structure 211a with a larger bending angle. The bent structure 211a of each load arm 20 is configured so that all the load arms 20 can be moved to a gathered state when in the non-working state. In some embodiments, there may be an odd number of load arms 20, where a midline of a certain load arm is taken as a symmetry axis, and the certain load arm may include or not include a bent section, while in every two adjacent load arms of the remaining load arms on both sides of the certain load arm, a load arm farther away from the midline has a larger bending angle.

In an embodiment, referring to FIG. 2 and FIG. 3, each load arm 20 is coupled to one setup arm 210. Referring to FIG. 4 and FIG. 5, the setup arm 210 may be coupled to the straight section 221 of the load arm 20, and may actively or passively move along the straight section 221 of the load arm 20. The movement of the setup arm 210 along the straight section 221 of the load arm 20 is limited, and all setup arms 210 have the same minimum radius and the same maximum radius of rotation about the rotation center or rotation axis AX1. All the setup arms 210 should move in the same annular region R. That is, within the movable range defined on the corresponding load arm 20 for each setup arm, all the setup arms have the same minimum distance R2 from the rotation axis AX1 and the same maximum distance R1 from the rotation axis AX1. Each setup arm moves in the annular region R between the minimum distance and the maximum distance, and this movement may be either a horizontal movement of the setup arm relative to the load arm, or a rotational movement of the setup arm driven by the load arm. In some embodiments, defining the movable range of the setup arms as the annular region R not only limits the motion range of the manipulator arms 220 coupled to the setup arms 210 within a certain space, thus satisfying usage requirements while reducing the free redundancy of the surgical robot, but also facilitates more favorable mechanical motion control of the manipulator arms 220.

In an embodiment, referring to FIG. 2 and FIG. 6, the load arms 20 are located at the same height of the orientation platform 10, where rotation planes of at least two load arms 20 are coplanar, and the coplanar plane is the same horizontal plane. In FIG. 2, four load arms 20 are disposed on the orientation platform 10, and have coplanar rotation planes, with the rotation centers of the four load arms 20 rotating relative to the orientation platform 10 still located on the same rotation axis AX1.

In FIG. 6, three load arms 20 are disposed on the orientation platform 10, and have coplanar rotation planes, with the rotation centers of the three load arms 20 rotating relative to the orientation platform 10 still located on the same rotation axis AX1.

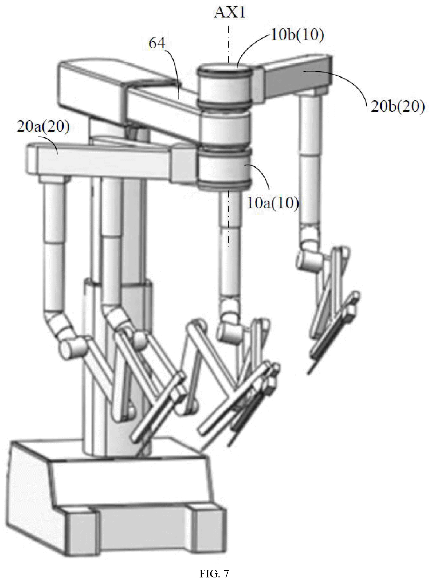

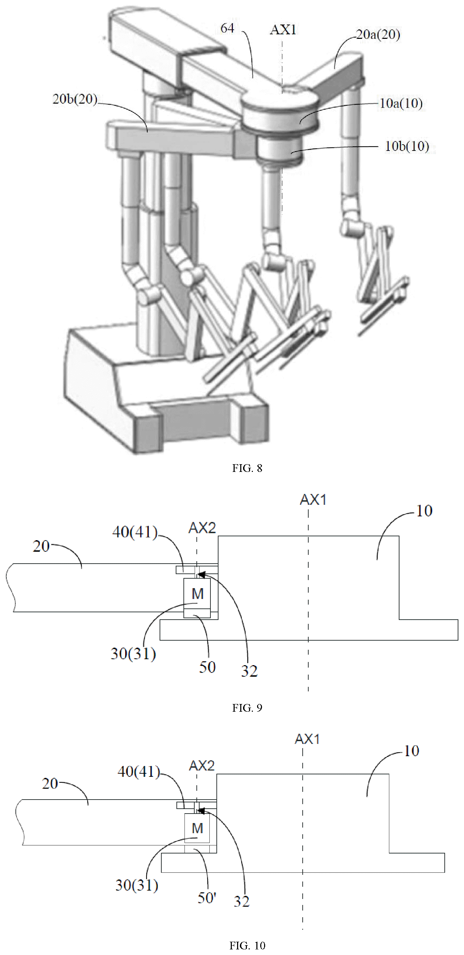

In an embodiment, as shown in FIG. 7 and FIG. 8, the load arms 20 are located at different heights of the orientation platform 10, where rotation planes of at least two load arms 20 are not coplanar, and are horizontal planes at different heights.

In FIG. 7, the orientation platform 10 includes two parts in a height direction, and radii of the two parts may be the same. In the illustrated example, the two parts of the orientation platform 10 are respectively located above and below an extendable boom member 64, and may have the same radius. A first part 10a of the orientation platform 10 located below the extendable boom member 64 is coupled to three load arms 20a, and a second part 10b of the orientation platform 10 located above the extendable boom member 64 is coupled to one load arm 20b, so that a rotation plane of the one load arm 20b coupled to the second part 10b and rotation planes of the three load arms 20a coupled to the first part 10a are not coplanar, and located at different heights. Rotation centers of these four load arms 20 rotating relative to the orientation platform 10 are still located on the same rotation axis AX1.

As shown in FIG. 8, the orientation platform 10 includes two parts in the height direction. In the illustrated example, the orientation platform 10 has a first part 10a located at an end of the extendable boom member 64, and a second part 10b located below the first part 10a, where a radius of the first part 10a may be different from a radius of the second part 10b. The second part 10b is coupled to three load arms 20b, and the first part 10a is coupled to one load arm 20a. A rotation plane of the one load arm 20a coupled to the first part 10a and rotation planes of the three load arms 20b coupled to the second part 10b are not coplanar, and located at different heights. Rotation centers of these four load arms 20 rotating relative to the orientation platform 10 are still located on the same rotation axis AX.

In combination with the above embodiments, as shown in FIG. 6 to FIG. 8, the load arms 20 are located at different heights of the orientation platform 10, and correspondingly, rotation planes of at least two load arms among at least three load arms 20 are coplanar.

In combination with the above embodiments, as shown in FIG. 6 to FIG. 8, the load arms 20 are located at different heights of the orientation platform 10, and rotation planes of at least two load arms among at least three load arms 20 are not coplanar.

In an embodiment, referring to FIG. 4 and FIG. 5, the surgical robot 200 includes a guide rail 11 fixed to the orientation platform 10. The guide rail 11 is configured as a complete annular structure or a partially annular structure, with its arc center located on the rotation axis AX1.

The guide rail 11 is a guiding component for the load arm 20 to rotate relative to the orientation platform 10. The guide rail 11 may be an integral component with the orientation platform 10, or may be a separate component mounted on the orientation platform 10. The guide rail 11 may be configured as a complete annular structure, so that the load arm 20 can rotate about the orientation platform 10 by an angle exceeding 360 degrees. The guide rail may also be configured as a partially annular structure, and the load arm 20 may rotate about the partially annular structure.

In an embodiment, referring to FIG. 2 and FIG. 6, the rotations of all the load arms 20 of the surgical robot 200 are coplanar, and all the load arms 20 may share the same guide rail.

When one guide rail is provided, the guide rail may be configured as a complete annular structure, or may be configured as a partially annular structure.

In an embodiment, referring to FIG. 7 and FIG. 8, the surgical robot 200 has at least two load arms 20 whose rotations are not coplanar, and the at least two load arms 20 are mounted on two different guide rails, and are each movable along a corresponding guide rail coupled thereto.

Referring to FIG. 9 to FIG. 12, in the embodiments of the present disclosure, the surgical robot may further include an active driving device 30/30'. For example, the active driving device 30/30' may be configured as a motor, or a combination of a motor and a reduction assembly. The active driving device 30/30' may include an active device body 31/31' and an active device output end 32/32'. The active driving device 30 may be disposed on the load arm 20, and the active device output end 32 may be engaged with the orientation platform 10 through a transmission device 40. The active driving device 30 can rotate together with the load arm 20 about the rotation axis AX1. The active driving device 30' may also be disposed on the orientation platform 10, and the active device output end 32' of the active driving device 30' is engaged with the load arm 20 through a transmission device 40', so the active driving device 30' does not rotate together with the load arm 20, but can drive the load arm 20 to rotate.

Specifically, in an embodiment, referring to FIG. 9 and FIG. 10, the active driving device 30 may be disposed on the load arm 20, the active device body 31 is fixed to the load arm 20, and the active device output end 32 rotates about its rotation axis AX2. The transmission device 40 includes a transmission input end 41 and a transmission output end, the transmission input end 41 is actuated by the active device output end 32, and the transmission output end is fixed to the orientation platform 10. For example, the transmission output end is fixed to an outer periphery of an annular structure of the orientation platform 10 (as in an example of gear meshing transmission described below), or the transmission output end at least includes a part fixed to the orientation platform 10 (as in an example described below where a transmission member such as a rope, a chain or a belt drives a roller for transmission). The active driving device 30 drives the transmission device 40 to drive the load arm 20 to rotate relative to the orientation platform 10. Further, in some embodiments, the transmission input end 41 is configured as a rotatable component, and the transmission output end is configured as a mating component that mates with the rotatable component. The active driving device 30 drives the mating component, thereby driving the rotation of the rotatable component, and then drives the load arm 20 to move along a circumferential direction of the orientation platform 10. Further, in some embodiments, the transmission input end or the rotatable component is configured as a pinion gear structure rotatably disposed on the load arm 20, and the pinion gear structure may be fixedly or transmissionally engaged with the active device output end 32. The transmission output end or the mating component may be configured as a large gear structure fixedly disposed on the orientation platform 10, and the pinion gear structure and the large gear structure form a meshing transmission relationship. In some embodiments, the transmission input end or the rotatable component may be configured as a roller structure, and the transmission output end or the mating component may be configured as a rope, chain, belt, or the like that drives the roller to rotate. In these embodiments, AX1 is the central axis of the orientation platform 10, and is also the rotation axis where the rotation centers of the load arms 20 are located; AX2 is the rotation axis of the active device output end 32' of the active driving device 30/30', and is also the rotation axis of the transmission input end 41 of the transmission device 40. In some embodiments, the rotation axis AX1 of the load arms 20 may be set parallel to the rotation axis AX2 of the active device output end.

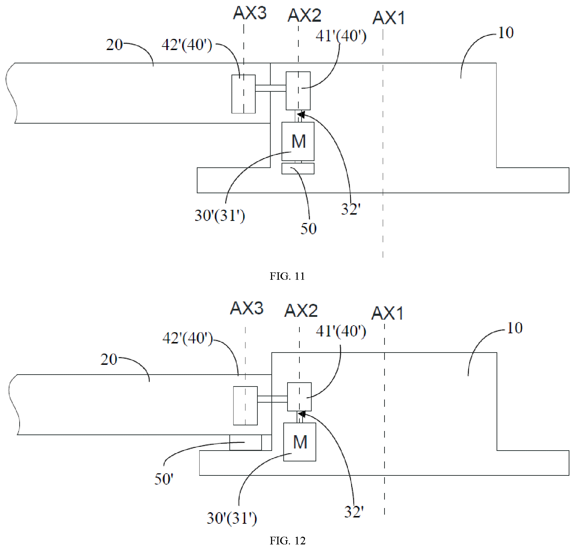

In another embodiment, referring to FIG. 11 and FIG. 12, the active driving device 30' may be disposed on the orientation platform 10. The active driving device 30' includes an active device body 31' and an active device output end 32'. The active device body 31' is fixed to the orientation platform 10, for example, fixed to an inner periphery of the annular structure of the orientation platform 10, and the active device output end 32' rotates about its rotation axis AX2. The transmission device includes a transmission input end 41' and a transmission output end 42'. The transmission input end 41' is actuated by the active device output end 32', and the transmission output end 42' is rotatably disposed on the load arm 20 about an axis AX3. The active driving device 30' drives the transmission device 40' to drive the load arm 20 to rotate relative to the orientation platform 10. In some embodiments, the transmission output end 42' is configured as a rotatable component, the transmission input end 41' is configured as a mating component that mates with the rotatable component, and the active driving device 30' drives the mating component, thereby driving the rotation of the rotatable component, and then drives the load arm 20 to move along the circumferential direction of the orientation platform 10. Further, in some embodiments, the transmission output end 42' or the rotatable component may be configured as a roller structure, and the transmission input end 41' or the mating component may be configured as a rope, chain, belt or the like that drives the roller to rotate. In these embodiments, AX1 is the central axis of the orientation platform 10, and is also the rotation axis where the rotation centers of the load arms 20 are located; AX2 is the rotation axis of the active device output end 32' of the active driving device 30', and is also the rotation axis of the transmission input end of the transmission device 40'; and AX3 is the rotation axis of the transmission output end of the transmission device 40'. In some embodiments, the rotation axis AX1 of the load arms 20 may be set parallel to the rotation axis AX3 of the transmission output end 42' of the transmission device 40.

In some embodiments, each of at least two load arms 20 is engaged with an active driving device and a transmission device.

In an embodiment, each of the at least two load arms 20 is engaged with an active driving device 30, and the active driving device 30 may be configured as a motor.

In an embodiment, at least two load arms of at least three load arms 20 share the same active driving device. The shared active driving device may be disposed on the orientation platform, and is engaged with the at least two load arms through a transmission device.

In some embodiments, the load arm 20 may not be engaged with an active driving device, and may be driven passively, for example, pushed by external force.

Referring to FIG. 9 to FIG. 12, in the embodiments of the present disclosure, the surgical robot may further include a braking device 50/50'. The braking device 50/50' may be coupled between the active driving device 30/30' and the load arm 20, and can brake the active driving device 30/30'. The braking device 50/50' may also be coupled between the load arm 20 and the orientation platform 10, and can brake the load arm 20. The braking device may also be coupled between the active driving device and the orientation platform. The braking device 50 can prevent the load arm 20 from rotating relative to the orientation platform 10, playing a locking role. After the adjustment is completed in the deployment stage of the surgical robot 200, the braking device 50 provides a relatively stable operating environment for the load arm 20, and is not unlocked unless necessary during the surgery. The braking device 50 may include a braking movable part and a braking fixed part.

Specifically, in some embodiments, referring to FIG. 9 and FIG. 11, the braking device 50 may be coupled to the active driving device 30/30', and can brake the active driving device. In the embodiment of FIG. 9, the braking movable part may be fixedly coupled to the active device output end 32 of the active driving device 30, and the braking fixed part may be coupled to the load arm 20. In the embodiment of FIG. 11, the braking movable part may be fixedly coupled to the active device output end 32 of the active driving device 30, and the braking fixed part is fixedly coupled to the orientation platform 10.

In other embodiments, the braking device may not be coupled to the active driving device. Referring to FIG. 10 and FIG. 12, the braking movable part may be fixedly coupled to the load arm 20, and the braking fixed part may be fixedly coupled to the orientation platform 10.

In an embodiment, at least two load arms 20 of at least three load arms 20 may share the same braking device 50.

In an embodiment, referring to FIG. 2, the surgical robot 200 further includes a support device 60, and the orientation platform 10 is disposed on the support device 60.

The support device 60 may include a fixed column 61, a lifting column 62, a fixed boom member 63, and an extendable boom member 64. The orientation platform 10 is coupled to the extendable boom member 64. The orientation platform 10 may be fixedly coupled to the extendable boom member 64 or movably disposed relative to the extendable boom member.

In an embodiment, the support device 60 includes a fixed column 61 and a lifting column 62, where the fixed column 61 is fixed to a mobile chassis 65, and the lifting column 62 is coupled to the fixed column 61 and is translationally movable relative to the fixed column 61 in a vertical direction. A screw mechanism may be provided inside the lifting column 62 to achieve extendable movement, or a pneumatic mechanism or a hydraulic mechanism may be provided inside the lifting column 62 to achieve extendable movement.

In an embodiment, the support device further includes a fixed boom member 63 and an extendable boom member 64, where the fixed boom member 63 is disposed at a top end of the lifting column 62, and the extendable boom member 64 is coupled to the fixed boom member 63 and is translationally movable relative to the fixed boom member 63 in a horizontal direction. A screw mechanism may be provided inside the extendable boom member 64 to achieve extendable movement, or a pneumatic mechanism or a hydraulic mechanism may be provided inside the extendable boom member 64 to achieve extendable movement.

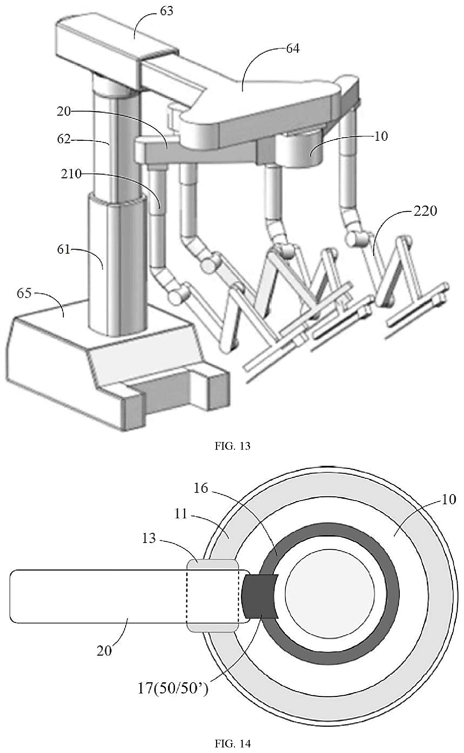

It should be noted that, referring to FIG. 13, the extendable boom member 64 in FIG. 13 may be configured as a Y-shaped or T-shaped structure, and the orientation platform 10 is translationally movable along an end of the extendable boom member 64, thereby expanding a movement range of the setup arms 210 and the manipulator arms 220.

Referring to FIG. 2, the surgical robot 200 may further include the setup arms 210 and the manipulator arms 220. As described above, the setup arm 210 is coupled to the straight section 221 of the load arm 20, and may actively or passively move along the straight section of the load arm 20. The setup arm 210 may telescope in the vertical direction, and a screw mechanism may be provided inside the setup arm 210 to achieve extendable movement. The manipulator arm 220 is coupled to a distal joint of the setup arm 210.

Through this arrangement, a mechanical transmission chain can be shortened, and the mechanical arm obtains relatively high relative stiffness.

As shown in FIG. 3, FIG. 14 and FIG. 15, since the orientation platform 10 is configured as an annular component or a component having an annular structure, so that the orientation platform 10 has an arc-shaped or circular outer peripheral surface 12. Each mechanical arm is individually coupled to the orientation platform 10 and is rotatable relative to the orientation platform 10, so as to adjust the position of the mechanical arm by rotating about the orientation platform 10. The rotation center of each mechanical arm rotating relative to the orientation platform 10 is located on a first axis AX1.

By providing the orientation platform 10, the degrees of freedom of the mechanical arms are simplified, structural redundancy is avoided, the overall convenience of using the surgical robot 200 is improved, the control program of the mechanical arms is easy to set, the control logic is clear, and the accuracy of performing surgery is improved.

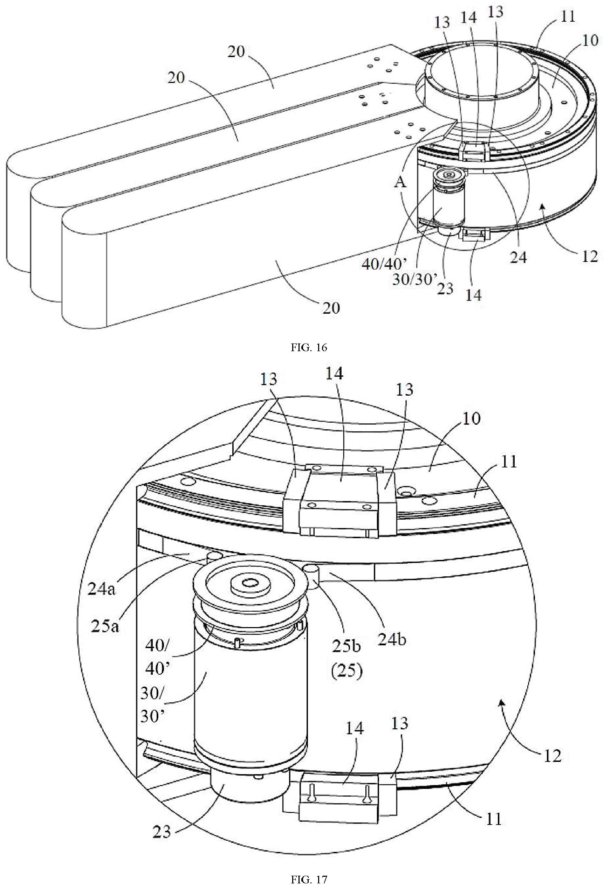

The orientation platform 10 includes a guide rail 11 fixed to the orientation platform 10. The mechanical arm may be coupled to the orientation platform 10 through the guide rail 11. Specifically, the operating arm 20 of the mechanical arm is coupled to the guide rail 11, thereby connecting the entire mechanical arm to the orientation platform 10. Exemplarily, as shown in FIG. 16 and FIG. 17, the guide rail 11 is provided with sliding blocks 13 and a mounting base 14. The mounting base 14 is coupled to the guide rail 11 through the sliding blocks 13 on both sides of the mounting base 14, and the operating arm 20 is coupled to the mounting base 14. Both the sliding blocks 13 and the mounting base 14 may be located inside the operating arm 20 so as to be hidden within the operating arm 20. In an embodiment, the guide rail 11 may include dual guide rails respectively disposed at an upper part and a lower part of the orientation platform 10. A top wall of the operating arm 20 is coupled to the guide rail 11 located at the upper part of the orientation platform 10, and a bottom wall of the operating arm 20 is coupled to the guide rail 11 located at the lower part of the orientation platform 10.

The guide rail 11 is a guiding component for the operating 20 to rotate relative to the orientation platform 10. The guide rail 11 may form an integral component with the orientation platform 10, or may be a separate component mounted on the orientation platform 10. The guide rail 11 may be configured as a complete annular structure, so that the operating arm 20 can rotate about the orientation platform 10 by an angle exceeding 360 degrees. Alternatively, the guide rail 11 may be configured as a partially annular structure, so that the operating arm 20 can rotate about the partially annular structure by an angle less than 360 degrees. As shown in FIG. 14, the operating arm 20 may be coupled with a braking device 50/50' including a braking stator 16 and a braking mover 17. All braking devices 50/50' may share one braking stator 16, and each load arm 20 is coupled with a respective braking mover 17. The braking stator 16 may be an annular stator concentric with the annular guide rail. The respective braking mover 17 is disposed on the load arm 20, and can directly brake the load arm, thereby maximally avoiding the problem of backlash clearance.

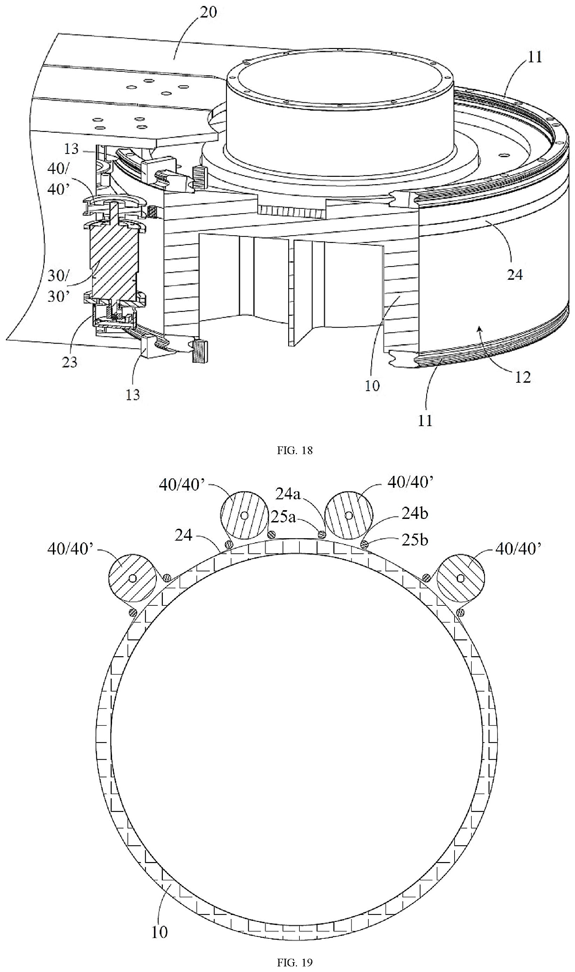

As shown in FIG. 16 and FIG. 17, the central axis of the annular structure of the guide rail 11 is defined as the first axis AX1, and a central axis of an arc or circle of the outer peripheral surface 12 of the orientation platform 10 may coincide with the first axis AX1. The operating arm 20 coupled to the guide rail 11 is rotatable about the first axis AX1 relative to the orientation platform 10. In order to realize the rotation of the operating arm 20 relative to the orientation platform 10, the operating arm 20 is provided with an active driving device 30/30' and a transmission device 40/40' actuatable by the active driving device 30/30'. The transmission device 40/40' may rotate about a second axis AX2 by being actuated by the active driving device 30/30', where the second axis AX2 is parallel to the first axis AX1. The transmission device 40/40' is disposed on an output shaft of the active driving device 30/30'. Exemplarily, the active driving device 30/30' and the transmission device 40/40' are both located inside the operating arm 20 and supported on a wall of the operating arm 20. A bottom of the active driving device 30/30' is provided with a base 23 (see FIG. 18), and the active driving device 30/30' is mounted to a bottom wall of the operating arm 20 through the base 23. The active driving device 30/30' may be, for example, a motor, and the transmission device 40/40' may be, for example, a rotating wheel.

The surgical robot 200 further includes a transmission member 24 configured to transmit a force of the transmission device 40/40' to the operating arm 20. The transmission member 24 may pass around the transmission device 40/40' and extend along the outer peripheral surface 12 of the orientation platform 10. The outer peripheral surface 12 of the orientation platform 10 is a smooth surface, and the transmission member 24 is in a tensioned state. With a frictional force between the transmission member 24 and the orientation platform 10, the transmission member 24 can be maintained at a desired position of the orientation platform 10, preventing the transmission member 24 from sliding up and down. If needed and/or desired, the outer peripheral surface 12 of the orientation platform 10 may be provided with a limiting structure for the transmission member 24, so as to limit the movement of the transmission member 24 in a height direction. An example of the limiting structure is a groove extending in the circumferential direction and configured to receive the transmission member 24.

The transmission member 24 at least includes a part fixed to the orientation platform 10. The illustrated example shows that the transmission member 24 is a closed member sleeved on the orientation platform 10. The transmission member 24 of this solution is suitable for the case where the orientation platform 10 has a circular outer peripheral surface 12. The transmission member 24 can go around the orientation platform 10 for one circle, so that the rotation angle of the load arm 20 around the orientation platform 10 can exceed 360 degrees. Alternatively, if there is no requirement for full-circle rotation, the transmission member 24 is an open member with its ends fixed to the orientation platform 10. The transmission member 24 of this solution is suitable for the case where the orientation platform 10 has an arc-shaped outer peripheral surface 12. The transmission member 24 goes around the orientation platform 10 for less than one circle, so that the rotation angle of the load arm 20 around the orientation platform 10 is less than 360 degrees.

The transmission device 40/40' rotates about the second axis AX2 while revolving about the first axis AX1, and drives the load arm 20 to rotate relative to the orientation platform 10 along the guide rail 11. The load arms 20 are guided by the guide rail 11 to move along the circumferential direction of the orientation platform 10, so that the load arms 20 are positioned at locations along the circumference of the orientation platform 10 when in operation, and gathered at one place of the orientation platform 10 when not in operation. In the present disclosure, the degrees of freedom of the load arms 20 can be simplified, and the overall convenience of use can be improved. Moreover, the load arm 20 is provided with a driving power, which can shorten the mechanical transmission chain, and the mechanical arm has a relatively high relative stiffness.

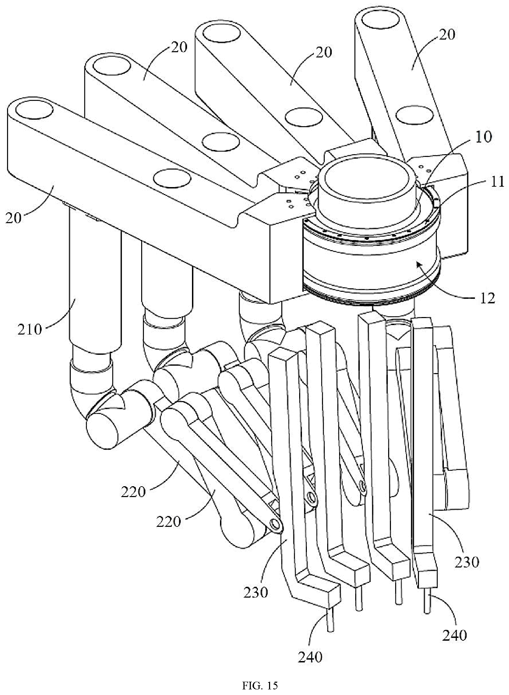

There are at least two load arms 20 coupled to the orientation platform 10. The at least two load arms 20 can be located on the same reference plane perpendicular to the first axis, or can be located at the same height of the orientation platform 10. Rotation planes of the at least two load arms 20 are coplanar, and the coplanar plane is the same horizontal plane. The at least two load arms 20 are movably coupled to the same guide rail 11. FIG. 15 schematically shows four load arms 20 coupled to the orientation platform 10, where rotation planes of the four load arms 20 are coplanar, and the four load arms 20 all rotate about the first axis AX1 relative to the orientation platform 10.

FIG. 16 to FIG. 19 schematically show an embodiment of the mechanical motion structure. As shown in FIG. 16 to FIG. 19, the transmission member 24 may be a belt. In this case, the transmission device 40/40' may be a belt pulley, and the outer peripheral surface 12 of the orientation platform 10 serves as a belt pulley. The belt has a certain width, and a surface in a thickness direction of the belt can be attached to the outer peripheral surface 12 of the orientation platform 10, so that a larger frictional force exists between the belt and the outer peripheral surface 12 of the orientation platform 10, preventing the belt from sliding up and down. The belt is wound on the outer peripheral surface 12 of the orientation platform 10 and passes around the transmission device 40/40'. In an embodiment, the belt is a metal belt, such as a steel belt. Alternatively, the belt is a synchronous belt. The belt may be a complete closed belt. Alternatively, if there is no requirement for full-circle rotation, the belt may be an open belt, that is, having two ends.

In the embodiment of the belt, at least one of the transmission member 24 and the transmission device 40/40' is provided with a plurality of teeth, so that the transmission member 24 and the transmission device 40/40' achieve transmission through tooth meshing. An example is that the transmission member 24 is provided with a plurality of holes spaced apart from each other, and the transmission device 40/40' is provided with a plurality of teeth spaced apart from each other, so that transmission between the transmission member 24 and the transmission device 40/40' is achieved through tooth-hole meshing. Alternatively, the transmission member 24 is provided with a plurality of teeth spaced apart from each other, and the transmission device 40/40' is also provided with a plurality of teeth spaced apart from each other, so that transmission between the transmission member 24 and the transmission device 40/40' is achieved through tooth-tooth meshing. A specific example is that the belt is provided with a plurality of holes, and the belt pulley is provided with a plurality of teeth spaced apart from each other. Alternatively, the belt is provided with a plurality of teeth, and the belt pulley is also provided with a plurality of teeth spaced apart from each other.

The belt transmission is achieved between the transmission member 24 and the transmission device 40/40' through tooth meshing, which can keep the transmission member 24 and the transmission device 40/40' moving synchronously, thereby controlling the rotation of the load arm 20 more accurately. When the transmission device 40/40' is actuated to rotate, under the action of the tooth meshing transmission, the transmission device 40/40' is actuated by the transmission member 24 to rotate about the second axis while revolving about the first axis AX1, thereby causing the load arm 20 to advance and stop.

As shown in FIG. 17 and FIG. 19, the load arm 20 may be provided with two adjusting wheels 25 (i.e., a first adjusting wheel 25a and a second adjusting wheel 25b) for a respective transmission device 40/40'. The two adjusting wheels 25 are, for example, rotatably coupled to a top wall of the load arm 20, are in contact with the transmission member 24, and are rotatable about their own respective third axes AX3. The two adjusting wheels 25 can be located between the transmission device 40/40' and the outer peripheral surface 12 of the orientation platform 10, and are spaced apart from each other along the circumferential direction of the orientation platform 10. The transmission member 24 passes between the two adjusting wheels 25. Specifically, the transmission member 24 includes a first transmission part 24a and a second transmission part 24b located on two sides of the transmission device 40/40' in the circumferential direction of the orientation platform 10. The first transmission part 24a is located between the two adjusting wheels 25 and passes around the first adjusting wheel 25a which is on the same side as the first transmission part 24a, and the second transmission part 24b is located between the two adjusting wheels 25 and passes around the second adjusting wheel 25b which is on the same side as the second transmission part 24b. The third axis is parallel to the second axis. The two adjustment wheels 25 allow adjustment of a wrap angle of the transmission member 24 at the transmission device 40/40', thereby preventing an excessively large wrap angle from affecting the movement space of adjacent load arms 20.

At least one of the two adjusting wheels 25 on the load arm 20 is movably provided so that a distance between the two adjusting wheels 25 is adjustable. Thus, the positions of the adjusting wheels 25 are variable, which enables fine adjustment of a tension degree of the transmission device 40/40' and adjustment of a wrap angle of the transmission device 40/40' at the transmission device 40/40'. In an embodiment, one of the two adjusting wheels 25 on the load arm 20 is movably provided.

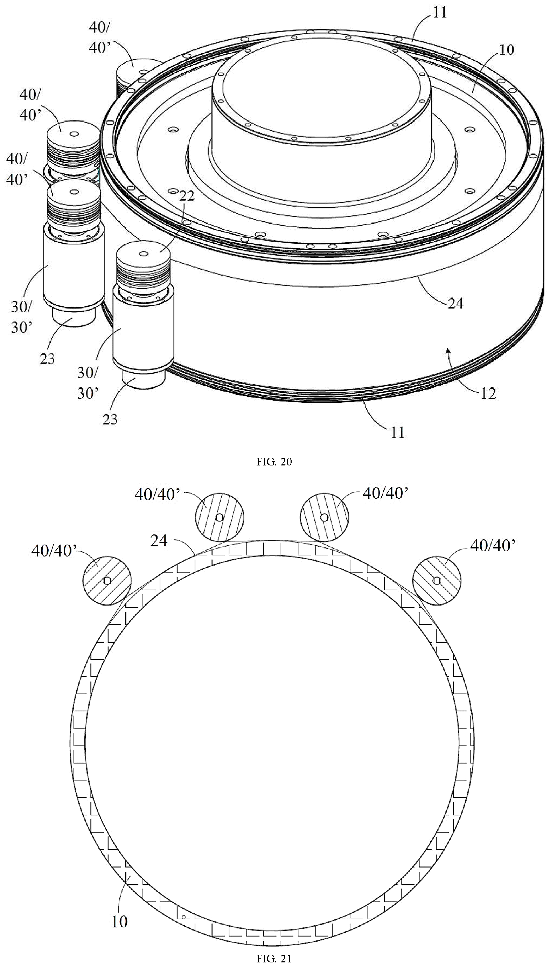

FIG. 20 and FIG. 21 schematically show another embodiment of the mechanical motion structure. As shown in FIG. 20 and FIG. 21, the transmission member 24 may be a rope. In this case, the transmission device 40/40' may be a winding wheel, and the outer peripheral surface 12 of the orientation platform 10 serves as a winding wheel. The rope is attached to the outer peripheral surface 12 of the orientation platform 10, so that a frictional force exists between the rope and the outer peripheral surface 12 of the orientation platform 10, preventing the rope from sliding up and down. The rope is wound on the outer peripheral surface 12 of the orientation platform 10 and passes around the transmission device 40/40'. In an embodiment, the rope is a metal rope, such as a steel wire rope. The rope may be wound on the orientation platform 10 for at least one circle. Alternatively, if there is no requirement for full-circle rotation, the rope is wound on the orientation platform 10 for less than one circle, and two ends of the rope are fixed to the orientation platform 10. When the transmission member 24 is a rope, the rope needs to be wound on the transmission device 40/40' for multiple circles, so as to increase the frictional force between the rope and the transmission device 40/40'.

In the embodiment of the rope, rope transmission is achieved between the transmission member 24 and the transmission device 40/40'. When the transmission device 40/40' is actuated to rotate, under the action of the frictional force between the rope and the transmission device 40/40', the transmission device 40/40' is actuated by the transmission member 24 to rotate about the second axis while revolving about the first axis AX1, thereby causing the load arm 20 to advance and stop. In an embodiment, two ropes are wound on the transmission device 40/40', and two ends of each rope are respectively coupled to the transmission device 40/40' and the orientation platform 10. Winding directions of the two ropes on the transmission device 40/40' are opposite. When the transmission device 40/40' rotates relative to the orientation platform 10, one of the two ropes is tightened and the other of the two ropes is released on the transmission device 40/40'. With this arrangement, rotation of the rotating member is easier.

Alternatively, the transmission member 24 may be a chain. In this case, the rotating member may be a sprocket, and the outer peripheral surface 12 of the orientation platform 10 serves as a sprocket. Through chain transmission, when the rotating member is actuated to rotate, under the action of a meshing force between the chain and the sprocket, the rotating member is actuated by the transmission member 24 to rotate about the second axis while revolving about the first axis, thereby causing the load arm 20 to advance and stop.

An example of the arrangement of the transmission member 24 is that the rotating member of all the load arms 20 are disposed at the same height, and one transmission member 24 passes around the rotating member of all the load arms 20. All the load arms 20 share one transmission member 24. In other words, one transmission member 24 connects the rotating member of all the load arms 20 in series. For the embodiments shown in FIG. 16 to FIG. 19, one belt passes around the rotating member of all the load arms 20, and exemplarily, one belt passes around the rotating member' of four load arms 20. For the embodiments shown in FIG. 20 and FIG. 21, one rope passes around the rotating member of all the load arms 20, and exemplarily, one rope passes around the transmission devices of four load arms 20. With this arrangement, independent rotation of multiple load arms 20 relative to the orientation platform 10 can be realized by using a single transmission member 24, featuring compact structure, and the transmission member 24 can be tensioned at one time.

Another example of the arrangement of the transmission member 24 is that the number of the transmission members 24 is the same as the number of the load arms 20. The transmission members 24 are staggered in the height direction, and one transmission member 24 passes around a rotating member of a corresponding one load arm 20. Positions of the rotating member in the height direction correspond to positions of the corresponding transmission members 24 in the height direction. That is, the rotating member are also staggered in the height direction. Exemplarily, four load arms 20 and four belts, ropes or chains are provided, and one belt, rope or chain passes around the rotating member of a corresponding one load arm 20. With this arrangement, each load arm 20 can be independently rotated by a single transmission member 24, and independent rotation of multiple load arms 20 relative to the orientation platform 10 can be respectively achieved by multiple transmission members 24, thereby providing the robotic arms with greater operational freedom, decoupling, and modular maintainability.

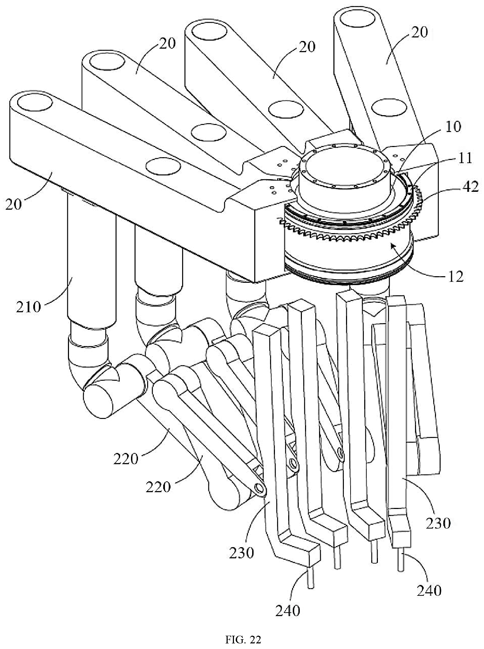

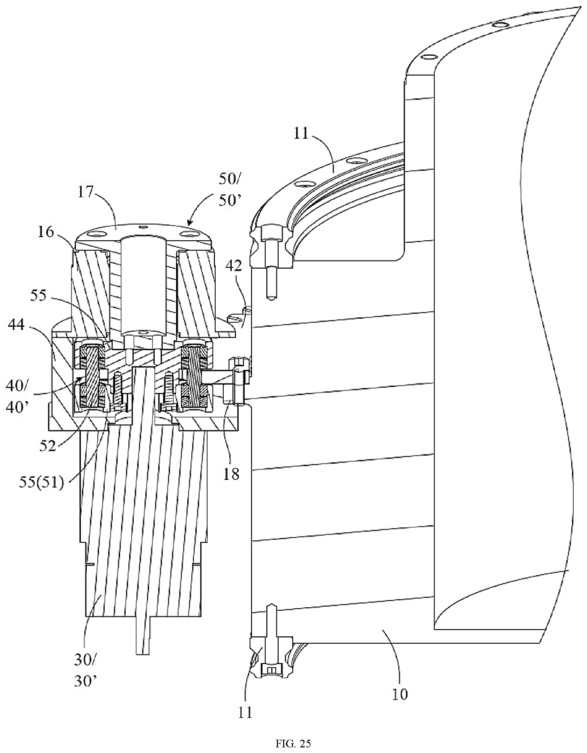

Referring to FIG. 22 and FIG. 25, in an embodiment, the transmission member includes a hypocycloidal gear ring 42 configured to transmit the force of the transmission device 40/40' to the load arm 20. The hypocycloidal gear ring 42 is disposed on the outer peripheral surface 12 of the orientation platform 10, for example, a boss 18 protruding from the outer peripheral surface 12 of the orientation platform 10 (see FIG. 25). The hypocycloidal gear ring 42 may be fixed to the boss 18 by fasteners such as screws. The hypocycloidal gear ring 42 can mesh with the transmission device 40/40'. A central axis of the hypocycloidal gear ring 42 may coincide with the first axis AX1. The illustrated example shows that the hypocycloidal gear ring 42 is a closed member sleeved on the orientation platform 10. The hypocycloidal gear ring 42 of this solution is suitable for the case where the orientation platform 10 has a circular outer peripheral surface 12, and the rotation angle of the load arm 20 around the orientation platform 10 can exceed 360 degrees. Alternatively, if there is no requirement for full-circle rotation, the hypocycloidal gear ring 42 is an open member. The hypocycloidal gear ring 42 of this solution is suitable for the case where the orientation platform 10 has an arc-shaped outer peripheral surface 12, and the rotation angle of the load arm 20 around the orientation platform 10 is less than 360 degrees.

The hypocycloidal gear ring 42 serves as a sun gear, and the transmission device 40/40' serves as a planetary gear. In this way, the mechanical motion structure between the load arm 20 and the orientation platform 10 can be configured as a planetary-like gear mechanism composed of the transmission device 40/40' and the hypocycloidal gear ring 42. When rotating, the transmission device 40/40' moves along a hypocycloidal path defined by the hypocycloidal gear ring 42, and drives the load arm 20 to rotate relative to the orientation platform 10 along the guide rail 11, and at this time, the load arm 20 can generate a yaw angle. The load arms 20 are guided by the guide rail 11 to move along the circumferential direction of the orientation platform 10, so that the load arms 20 are positioned at locations along the circumference of the orientation platform 10 when in operation, and gathered at one place of the orientation platform 10 when not in operation. In the present disclosure, the degrees of freedom of the load arm 20 can be simplified, the overall convenience of use can be improved, the transmission accuracy between the load arm and the orientation platform 10 can be improved, the transmission efficiency is high, and the transmission is smooth. Moreover, the load arm is provided with a driving power, which can shorten the mechanical transmission chain, and the mechanical arm has a relatively high relative stiffness.

Exemplarily, the transmission devices 40/40' of all the load arms 20 are disposed at the same height, and mesh with one hypocycloidal gear ring 42. All the load arms 20 share one hypocycloidal gear ring 42. In other words, one hypocycloidal gear ring 42 drivingly connects the transmission devices 40/40' of all the load arms 20. In the illustrated embodiment, one hypocycloidal gear ring 42 drivingly connects the transmission devices 40/40' of four load arms 20. With this arrangement, independent rotation of multiple load arms 20 relative to the orientation platform 10 can be realized by using a single hypocycloidal gear ring 42, featuring compact structure.

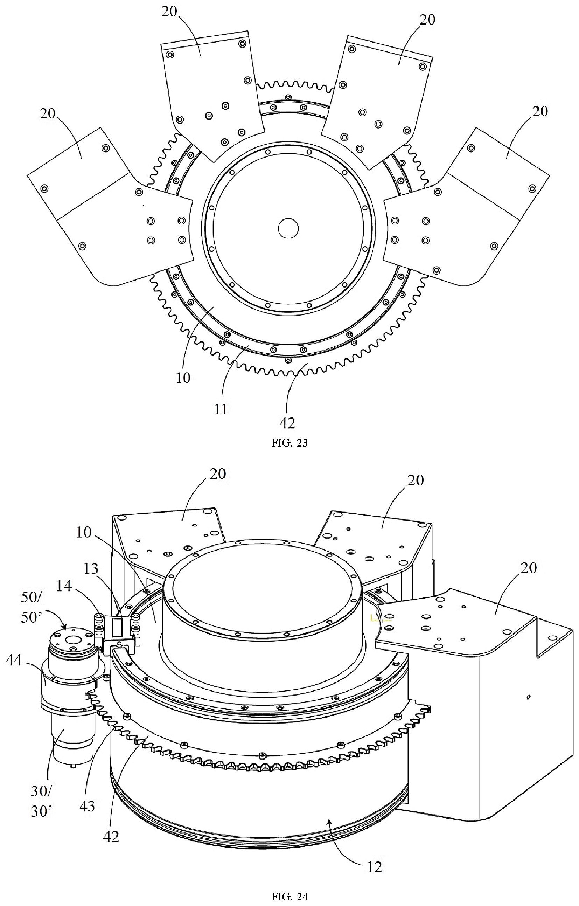

As shown in FIG. 24 and FIG. 25, the load arm 20 is provided with a support member 44, and the transmission device 40/40' is fixed to the active driving device 30/30' through the support member 44. The active driving device 30/30' is located on a lower side of the support member 44 and passes through the support member 44 to connect to the transmission device 40/40'. Thus, the active driving device 30/30' and the transmission device 40/40' can form a modular structure, which is convenient for maintenance. Each load arm 20 is provided with a braking device 50/50' configured to brake the transmission device 40/40' or the active driving device 30/30'. Disposing the braking device 50/50' on the load arm 20 in cooperation with a planetary-gear-like transmission enables stable travel and braking of the load arm 20, eliminating brake slack resulting from backlash of the teeth 43.

In the illustrated embodiment, the braking device 50/50' is configured to brake the transmission device 40/40'. The braking device 50/50' is located on an upper side of the transmission device 40/40' and is coupled to a rotating assembly 50. Specifically, the braking device 50/50' includes a braking stator 16 and a braking mover 17. The braking stator 16 is located on an upper side of the support member 44 and may be coupled to the support member 44. Specifically, a bottom of the braking stator 16 is provided with a flange having multiple through holes, and the flange is connected to the support member 44 by fasteners such as screws. The braking mover 17 is disposed on the braking stator 16 and is coupled to the transmission device 40/40'. Exemplarily, the braking stator 16 has a central hole, and the braking mover 17 is inserted into the central hole and coupled to the transmission device 40/40'. A top of the braking mover 17 is provided with a flange having multiple through holes, and the flange is connected to the braking stator 16 by fasteners such as screws.

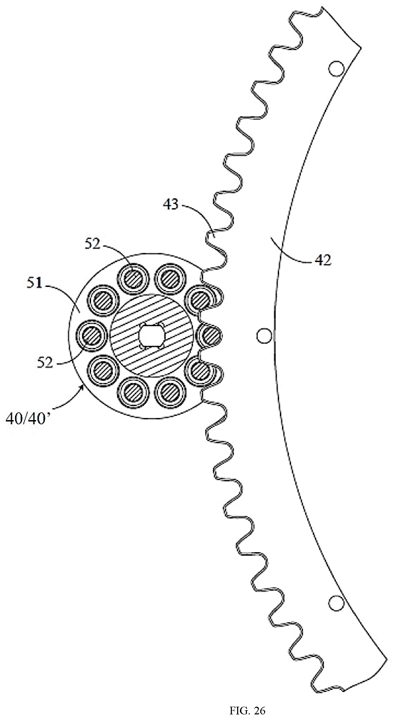

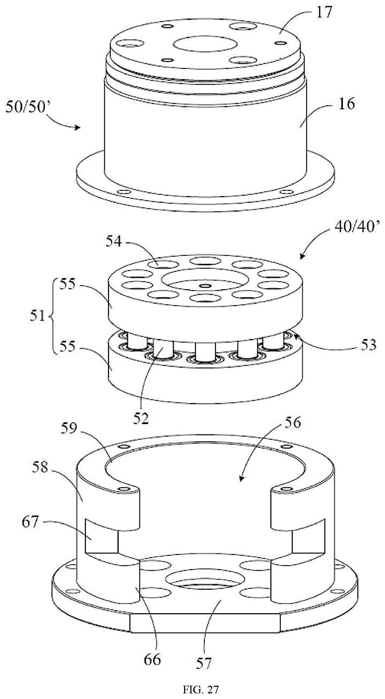

As shown in FIG. 26 and FIG. 27, the transmission device 40/40' may include a rotating wheel 51 and a plurality of rotating pins 52. The rotating wheel 51 is actuatable by the active driving device 30/30' to rotate about the second axis AX2. The plurality of rotating pins 52 are disposed on the rotating wheel 51 and are arranged at intervals along a circumferential direction defined by the second axis AX2. In an embodiment, the plurality of rotating pins 52 are in an annular array relative to the second axis AX2, and a distance between every two adjacent rotating pins 52 may be the same. The illustrated embodiment schematically shows ten rotating pins 52. Of course, the number of rotating pins 52 is not limited and can be set as needed.

Outer peripheral surfaces 12 of the rotating pins 52 can mesh with the teeth 43 of the hypocycloidal gear ring 42. Further, when the transmission device 40/40' meshes with the hypocycloidal gear ring 42, a tooth 43 of the hypocycloidal gear ring 42 moves into space between two adjacent rotating pins 52 and come into contact with the outer peripheral surfaces of the two adjacent rotating pins 52.

When the active driving device 30/30' drives the rotating wheel 51 to rotate, the plurality of rotating pins 52 also rotate with the rotating wheel 51. Since the rotating pins 52 mesh with the teeth 43 of the hypocycloidal gear ring 42, the transmission device 40/40' moves along the hypocycloidal path defined by the hypocycloidal gear ring 42, thereby driving the load arm 20 to rotate relative to the orientation platform 10 along the guide rail 11. The rotating wheel 51 is a fixing structure for the rotating pins 52, and does not directly mesh with the teeth 43 of the hypocycloidal gear ring 42; instead, the rotating pins 52 mesh with the teeth 43 of the hypocycloidal gear ring 42. This can further improve the transmission accuracy between the load arm 20 and the orientation platform 10.

The plurality of rotating pins 52 are rotatable about their own respective axes AX3 parallel to the second axis AX2, and coincide with central axes of the rotating pins 52 respectively. The rotatable rotating pins 52 can reduce mechanical friction between the rotating pins 52 and the hypocycloidal gear ring 42, avoiding a jamming phenomenon caused by mechanical friction, and making the transmission smoother.

The rotating wheel 51 is provided with a receiving groove 53. A groove opening of the receiving groove 53 is recessed from the outer peripheral surface 12 of the rotating wheel 51, thereby forming an annular groove. The rotating pin 52 passes through the receiving groove 53. Specifically, mounting holes 54 are opened on each of an upper side and a lower side of the receiving groove 53 of the rotating wheel 51, and can communicate with the receiving groove 53. The rotating pins 52 are movably inserted into the mounting holes 54 respectively and passes through the receiving groove 53. The teeth 43 of the hypocycloidal gear ring 42 can extend into the receiving groove 53 through the groove opening to mesh with the rotating pins 52. By providing the receiving groove 53 on the outer peripheral surface 12 of the rotating wheel 51, the teeth 43 of the hypocycloidal gear ring 42 can easily mesh with the rotating pins 52, featuring compact structure.

See FIG. 25 and FIG. 27, the rotating wheel 51 includes two wheel discs 55 detachably connected to each other. In an embodiment, middle portions of the two wheel discs 55 may be connected by fasteners such as screws. Of course, a connection mode of the two wheel discs 55 is not limited and may be set as needed. The receiving groove 53 is formed between the two wheel discs 55, and each wheel disc 55 is provided with the mounting holes 54. Two ends of the rotating pin 52 are respectively movably received in two mounting holes 54 of the two wheel discs 55. By constructing the rotating wheel 51 in a split manner, the rotatable rotating pins 52 can be easily assembled.

The support member 44 is provided with an accommodating cavity 56. The accommodating cavity 56 has an end wall 57, a side wall 58, an end opening 59 opposite to the end wall 57 in a direction parallel to the second axis, and a side opening 66 opened in the side wall 58. The rotating assembly 50 may enter the accommodating cavity 56 through the end opening 59 and be supported on the end wall 57. The end wall 57 may be connected to the active driving device 30/30' by fasteners such as screws, and the output end of the active driving device 30/30' passes through the end wall 57.

At least the teeth 43 of the hypocycloidal gear ring 42 extend into the accommodating cavity 56 through the side opening 66 to mesh with the transmission device 40/40'. As can be seen from FIG. 25, the teeth 43 and a main body of the hypocycloidal gear ring 42, as well as the boss 18 for mounting the hypocycloidal gear ring 42, all extend into the accommodating cavity 56 through the side opening 66. By providing the side opening 66 on an outer periphery of a supporting surface, the teeth 43 of the hypocycloidal gear ring 42 can easily mesh with the rotating pins 52, featuring compact structure. In an embodiment, the support member 44 is further provided with notches 67 on two sides of the side opening 66 in the circumferential direction. When the load arm 20 rotates, the teeth 43 enter the accommodating cavity 56 through the notches 67 to prepare for engaging with the transmission device 40/40' and preparing for disengaging from the transmission device 40/40'. With this arrangement, a size of the side opening 66 in the circumferential direction of the support member 44 can be made smaller, ensuring structural strength of the support member 44.

Alternatively, as shown in FIG. 14, the braking device 50/50' may not be disposed on the transmission device 40/40'. The orientation platform 10 is provided with a braking device 50/50' corresponding to the load arm 20, and the braking device 50/50' is coupled to the load arm 20 to brake the load arm 20. The braking stator 16 may be an annular stator concentric with the annular guide rail 11. The braking mover 17 is disposed on the braking stator 16 and is coupled to the end of the load arm 20. All braking devices 50/50' may share one braking stator 16, and each load arm 20 is coupled with a respective braking mover 17. Thus, the load arm can be directly braked, which can maximally avoid the problem of backlash clearance.

The surgical robot and the medical system provided by the embodiments of the present disclosure have been described in detail above. Specific examples are used herein to explain the principles and implementations of the present disclosure. The description of the above embodiments is only used to help understand the idea of the present disclosure. Changes may be made to the specific implementations and application scopes. In summary, the content of this specification should not be construed as limiting the present disclosure.

## Claims

1. A surgical robot, comprising:
an orientation platform; and
at least two load arms, coupled to the orientation platform and rotatable relative to the orientation platform about a same axis.

2. The surgical robot according to claim 1, further comprising:
at least two setup arms, each coupled to an associated load arm and movable relative to the associated load arm along an extension direction of the associated load arm, wherein the at least two setup arms have a same minimum radius and a same maximum radius of rotation about a central axis of the orientation platform.

3. The surgical robot according to claim 1 or claim 2, wherein each load arm of the at least two load arms includes a straight section, and straight sections of the at least two load arms are configured to be aligned side by side.

4. The surgical robot according to any one of claims 1 to 3, wherein rotation planes of the at least two load arms are coplanar; or
wherein rotation planes of the at least two load arms are not coplanar; or
wherein at least three load arms are provided, and rotation planes of at least two load arms of the at least three load arms are coplanar; or
wherein at least three load arms are provided, and rotation planes of at least two load arms of the at least three load arms are not coplanar.

5. The surgical robot according to any one of claims 1 to 4, further comprising:
a guide rail, fixed to the orientation platform and configured as a complete annular structure or a partially annular structure.

6. The surgical robot according to any one of claims 1 to 5, further comprising:
one guide rail, wherein the at least two load arms are coupled to the guide rail and movable along the guide rail; or
at least two guide rails, wherein the at least two load arms are coupled to the at least two guide rails, and each of the at least two load arms is movable along a corresponding guide rail coupled thereto.

7. The surgical robot according to any one of claims 1 to 6, wherein each load arm of the at least two load arms is engaged with an active driving device including an active device body and an active device output end;
wherein the active device body is fixed to the associated load arm or the orientation platform.

8. The surgical robot according to any one of claims 1 to 7, wherein:
each respective load arm of the at least two load arms is engaged with an active driving device and a transmission device, the active driving device includes an active device body and an active device output end, the active device body is fixed to the respective load arm, the active device output end is rotatable about its axis, the transmission device includes a transmission input end and a transmission output end, the transmission input end is actuatable by the active device output end, the transmission output end is fixed to the orientation platform, and the active driving device is configured to actuate the transmission device to drive rotation of the respective load arm relative to the orientation platform; or
each respective load arm of the at least two load arms is engaged with an active driving device and a transmission device, the active driving device includes an active device body and an active device output end, the active device body is fixed to the orientation platform, the active device output end is rotatable about its axis, the transmission device includes a transmission input end and a transmission output end, the transmission input end is actuatable by the active device output end, the transmission output end is rotatably disposed on the respective load arm, and the active driving device is configured to actuate the transmission device to drive rotation of the respective load arm relative to the orientation platform.

9. The surgical robot according to any one of claims 1 to 8, wherein each load arm of the at least two load arms is engaged with a braking device; or
wherein some of the at least two load arms are engaged with a same braking device.

10. The surgical robot according to any one of claims 1 to 9, further comprising:
a support device, wherein the orientation platform is disposed on the support device;
wherein the orientation platform is fixed to the support device, or translationally movable along the support device.

11. The surgical robot according to claim 10, wherein the support device includes:
a fixed column, fixed to a mobile chassis;
a lifting column, coupled to the fixed column and translationally movable in a vertical direction relative to the fixed column;
a fixed boom member, disposed at a top end of the fixed column; and
an extendable boom member, coupled to the fixed boom member and translationally movable in a horizontal direction relative to the fixed boom member, wherein the orientation platform is disposed on the extendable boom member.

12. The surgical robot according to any one of claims 1 to 3, wherein:
the orientation platform has an outer peripheral surface in the shape of an arc or a circle, the orientation platform includes a guide rail configured as a complete annular structure or a partially annular structure, and a central axis of the annular structure of the guide rail is defined as a first axis which coincides with a central axis of the arc or the circle of the outer peripheral surface;
each respective load arm of the at least two load arms is coupled to the guide rail and is rotatable about the first axis relative to the orientation platform, the respective load arm is provided with an active driving device and a transmission device actuatable by the active driving device, and the transmission device is rotatable about a second axis parallel to the first axis;
the surgical robot further includes a transmission member passing around the transmission device and extending along the outer peripheral surface of the orientation platform, the transmission member at least includes a portion fixed to the orientation platform, and the transmission device is configured to rotate about the second axis while revolving about the first axis, so as to drive the respective load arm to move along the guide rail relative to the orientation platform.

13. The surgical robot according to claim 12, wherein two load arms of the at least two load arms are located on a same reference plane perpendicular to the first axis and are movably coupled to the guide rail.

14. The surgical robot according to claim 13, wherein the transmission devices of the at least two load arms are disposed at a same height, and the transmission member passes around the transmission devices of the at least two load arms.

15. The surgical robot according to claim 13, wherein a number of transmission members is the same as a number of the at least two load arms, the transmission members are staggered in a height direction, one respective transmission member of the transmission members passes around the associated transmission device of the associated load arm, and positions of the transmission devices in the height direction respectively corresponds to positions of the transmission members in the height direction.

16. The surgical robot according to any one of claims 12 to 15, wherein the respective load arm is provided with two adjusting wheels that are in contact with the transmission member and each rotatable about its own third axis, the two adjusting wheels are located between the transmission member and the outer peripheral surface of the orientation platform and are spaced apart from each other along a circumferential direction of the orientation platform, the transmission member passes between the two adjusting wheels, and the third axis is parallel to the second axis.

17. The surgical robot according to any one of claims 12 to 16, wherein at least one of the transmission member and the transmission device is provided with a plurality of teeth, so that transmission between the transmission member and the transmission device is achieved through tooth meshing.

18. The surgical robot according to claim 17, wherein the transmission member is provided with a plurality of holes spaced apart from each other, and the transmission device is provided with the plurality of teeth spaced apart from each other, so that the transmission between the transmission member and the transmission device through tooth-hole meshing; or
wherein the transmission member is provided with a first plurality of teeth spaced apart from each other, and the transmission device is provided with a second plurality of teeth spaced apart from each other, so that the transmission between the transmission member and the transmission device through tooth-tooth meshing.

19. The surgical robot according to any one of claims 12 to 16, wherein the transmission member includes at least one of a rope, a chain, and a belt, and the rope is wound around the transmission device in multiple turns; and/or
wherein the outer peripheral surface of the orientation platform is a smooth surface, or is provided with a limiting structure for the transmission member.

20. The surgical robot according to any one of claims 12 to 16, wherein the transmission member is a closed member sleeved on the orientation platform; or
wherein the transmission member is an open member having ends fixed to the orientation platform.

21. The surgical robot according to any one of claims 12 to 16, wherein the transmission member includes two ropes wound around the transmission device, both ends of each rope of the two ropes are respectively connected to the transmission device and the orientation platform, and winding directions of the two ropes on the transmission device are opposite.

22. The surgical robot according to claim 12, wherein:
the transmission member includes a hypocycloidal gear ring disposed on the outer peripheral surface of the orientation platform and configured to mesh with the transmission device, and the transmission device is configured to move along a hypocycloidal path defined by the hypocycloidal gear ring when rotating and to drive the respective load arm to move along the guide rail relative to the orientation platform.

23. The surgical robot according to claim 22, wherein the respective load arm is provided with a braking device configured to brake the transmission device or the active driving device.

24. The surgical robot according to claim 22 or claim 23, wherein transmission devices of the at least two load arms are disposed at a same height and configured to mesh with the hypocycloidal gear ring.

25. The surgical robot according to any one of claims 22 to 24, wherein the transmission device includes a rotating wheel and a plurality of rotating pins, the rotating wheel is actuatable by the active driving device to rotate about the second axis, the plurality of rotating pins are disposed on the rotating wheel and are arranged at intervals along a circumferential direction defined by the second axis, and outer peripheral surfaces of the plurality of rotating pins mesh with teeth of the hypocycloidal gear ring.

26. The surgical robot according to claim 25, wherein each of the plurality of rotating pins is rotatable about its third axis parallel to the second axis.

27. The surgical robot according to claim 25 or claim 26, wherein the rotating wheel is provided with a receiving groove, a groove opening of the receiving groove is recessed from the outer peripheral surface of the rotating wheel, the plurality of rotating pins pass through the receiving groove, and the teeth of the hypocycloidal gear ring are configured to extend into the receiving groove through the groove opening to mesh with the plurality of rotating pins.

28. The surgical robot according to claim 27, wherein the rotating wheel includes a first wheel disc and a second wheel disc detachably connected to each other, the receiving groove is formed between the first wheel disc and the second wheel, the first wheel disc is provided with first mounting holes, the second wheel disc is provided with second mounting holes, and two ends of each respective rotating pin of the plurality of rotating pins are respectively movably received in a respective first mounting hole of the first wheel disc and a respective second mounting hole of the second wheel disc.

29. The surgical robot according to any one of claims 22 to 28, wherein the respective load arm is provided with a support member having an accommodating cavity, the accommodating cavity has an end wall, an end opening opposite to the end wall, and a side opening, the transmission device is configured to enter the accommodating cavity through the end opening and is supported on the end wall, and at least the teeth of the hypocycloidal gear ring are configured to extend into the accommodating cavity through the side opening to mesh with the transmission device.

30. The surgical robot according to any one of claims 22 to 29, wherein the transmission device is fixed to the active driving device through the support member.

31. The surgical robot according to any one of claims 22 and 24 to 28, wherein the orientation platform is provided with a braking device corresponding to the respective load arm, and the braking device is coupled to the respective load arm to brake the respective load arm.

32. The surgical robot according to any one of claims 22 to 28, wherein the at least two load arms are located on a same reference plane perpendicular to the first axis and are movably coupled to the guide rail.

33. A medical system, comprising the surgical robot according to any one of claims 1 to 32.
